# EUROPEAN PATENT SPECIFICATION

(11) **EP 1 253 857 B1**
(45) Date of publication and mention of the grant of the patent: **21.01.2009**
(21) Application number: 01902554.3
(22) Date of filing: 05.02.2001
(51) Int. Cl.: A61B 17/00, A61L 17/08

(54) **DEVICE FOR THE CLOSURE OF A SURGICAL PUNCTURE**
GERÄT ZUM VERSCHLIESSEN EINER CHIRURGISCHEN PUNKTIONSWUNDE
DISPOSITIF PERMETTANT DE FERMER UNE PERFORATION CHIRURGICALE

(30) Priority: 03.02.2000 GB 0002378; 03.02.2000 GB 0002379
(43) Date of publication of application: 06.11.2002
(73) Proprietor: Tissuemed Limited, Leeds LS14 6UF (GB)
(72) Inventor: FORTUNE, David, Bramhope, Leeds LS16 9EU (GB); VELADA, Jose, Selby, North Yorkshire YO8 3WB (GB); TROTTER, Patrick, Leeds LS6 4RD (GB); MANDLEY, David, Melton Mowbray, Leicestershire LE14 3LU (GB); EDWARDSON, Peter, Alwoodley, Leeds LS17 8EB (GB)
(74) Representative: Jones, Stephen Anthony
(86) International application number: PCT/GB2001/000454
(87) International publication number: WO 2001/056475

(56) References cited:
- WO-A-00/59380
- WO-A-99/52481
- US-A- 4 744 364
- US-A- 5 312 435
- US-A- 5 571 181

## Description

This invention relates to devices intended for implantation in the body in the course of surgical procedures, and to a process for manufacture of such devices. The invention relates particularly to implantable devices useful in in the closure of a surgical puncture and manufacturable in a wide variety of forms suitable for many different applications.

WO 96/22797 discloses a tissue-bonding material comprising an aqueous albumin solution and a chromophore such as methylene blue. The material can be used to bond together tissues, eg the opposing edges of two blood vessels that are to be joined, by application of the material to one or both of those edges, followed by the bringing together of the tissues that are to be joined, and application of light energy to bring about cross-linking of the albumin to itself and to the tissues, thereby creating a bond. The methylene blue serves to facilitate the absorption of the light energy and also prevents excessive absorption of energy by undergoing a reversible colour change that stops energy being absorbed as well as signalling to the user that curing has been effected.

Our co-pending International patent application PCT/GB99/02717 discloses albumin-based sheets, which can be applied topically and caused to crosslink and bond to the underlying tissue. Though capable to a limited extent of being formed by the user into, for instance, tubes or rolls, such sheets are essentially two-dimensional structures and are therefore limited in their range of applications. Typically, such sheets are useful only as patches or the like applied to the external surface of a vessel such as an artery, eg to cover and close a puncture in that vessel. Even in these applications, however, the sheets may be of limited utility because in practice the degree of bonding between the sheet and the arterial tissue may be insufficient to withstand the associated pressures.

The implantation of devices within the body is commonplace in surgical procedures. Many such devices are known, and they are often manufactured from metallic or synthetic polymeric materials. A problem that may be encountered with such devices is that they can become dislodged from the site of application, leading to a failure of the device to perform its intended function, or more seriously to complications requiring further surgical intervention. Such problems may be addressed by attempting to fix the device securely in position, eg by the use of sutures or other forms of mechanical fastener, but this is often difficult to achieve.

We have now surprisingly found that tissue bonding material of the type known for use in liquid or planar sheet form can also be used to create pre-formed three-dimensional structures of use in the manufacture and use of implantable devices, and that such structures overcome or substantially mitigate the above-mentioned or other disadvantages of the prior art.

In many surgical procedures it is necessary to make a puncture in the relevant tissue or vessel, eg an artery may be punctured to enable the introduction of a surgical or other device. This gives rise to a need to close such a puncture, and this may not be easy to achieve.

The present invention provides a device which addresses this specific problem.

According to the invention, there is provided a pre-formed three-dimensional article in the form of a device for use in the closure of a surgical puncture, said device comprising a sheet of material which is flexible, hydratable and capable of bonding to tissue whilst retaining its integrity, which material may be activated, leading to cross-linking of the material and the formation of chemical bonds between the material and tissue to which it is applied, and/or which material is inherently self-adhesive; said sheet being folded or collapsed to a condition such that it can be passed through the puncture into the organ or vessel in which the puncture is formed, and said sheet being adapted to expand within the organ or vessel to an operative condition in which the sheet bears against the internal surface of the organ or vessel.

The article according to the invention is advantageous primarily in that it can be pre-forrmed in any of a range of shapes and forms appropriate to its intended application. Because the material from which the article is formed is capable of bonding to the surrounding tissue, the article can be securely anchored within that tissue, with reduced danger of the article becoming dislodged.

The article according to the invention may be attached to the surrounding tissue by one or more of a variety of methods. The material may be activated, eg by irradiation with light as described in more detail below, leading to cross-linking of the material (curing) and the formation of chemical bonds between the material and the tissue. Alternatively, the material may be inherently self-adhesive. In a further alternative, or for additional security in cases where it is possible to do so, the article may be secured by suturing. Combinations of some or all of these attachment methods may also be used.

The articles can be manufactured in such a way that they are expansible. They can be constructed in such a way as to be permanent, so that they retain their integrity and remain in place for an indefinite period. Alternatively, the articles can be manufactured in such a way as to be partially or wholly biodegradable so that they function for long enough to fulfil their intended purpose but then disintegrate.

The articles according to the invention may have a continuous or open structure. A continuous structure may be favoured where the article has a barrier function, eg to prevent formation of post-surgical adhesions. An open structure may be used where ingrowth of host tissue is desired, eg in vascular closure or where the article functions as a surgical mesh. The article may be partially biodegradable so that it initially serves as a barrier to tissue growth but then degrades to an open structure that supports tissue ingrowth.

The articles according to the invention may also act as a depot for the short- or long-term, localised or systemic delivery of pharmacologically active compounds (eg drugs for tumour reduction, cell growth inhibitors, antibiotics, anti-ulcer drugs etc), growth factors, bio-active polypeptides, proteins, antibodies or cells (eg fibroblasts, keratinocytes for wound healing and in the treatment of wounds).

The material used in the article according to the invention is preferably entirely tissue-compatible. The material is preferably also non-thrombogenic. The hydratable and activatable material is most commonly a crosslinkable proteinaceous or other peptide material. The material may be selected from natural and synthetic peptides, enzymatically cleaved or shortened variants thereof and crosslinked derivatives thereof, as well as mixtures of any of the above. Included among the peptides are structural proteins and serum proteins. Examples of proteins are albumin, α-globulins, β-globulins, γ-globulins, transthyretin, collagen, elastin and fibronectin and coagulation factors including fibrinogen, fibrin and thrombin. The preferred tissue-compatible material for use in the present invention is a soluble protein that is not part of the clotting cascade, such as albumin. Porcine albumin or porcine pericardium or any other abundant non-thrombogenic protein, ie excluding collagen, may be used. In some cases, genetically or chemically modified versions of these proteins may be used.

The material may also include one or more additional components to modify its physical properties. Such components may be elastomers or plasticisers, examples being polyalcohols such as glycerol, polyvinylalcohol and polyethyleneglycol.

It is particularly preferred that the hydratable tissue-bonding material of which the article is made up should comprise albumin in admixture with one or more other components. Mammalian albumin, especially porcine albumin, is especially preferred. Glycerol is a particularly preferred additional component.

In the folded or collapsed condition the sheet will generally have a configuration which permits the sheet to be passed through the surgical puncture. The sheet may, for instance, have an elongated, ovoid or rectangular shape and be folded about the lateral axis of the sheet. In another embodiment, the sheet may be generally circular and may be folded in the manner of a filter paper or the like, ie a fluted configuration such as that of a collapsed or partially collapsed umbrella.

To facilitate manipulation of the sheet of material it may be attached to a stem or rod, most preferably of a biocompatible material. The stem or rod is most preferably of a solid proteinaceous material, eg it may be albumin-based.

Opening of the sheet of material from the collapsed to the operative condition may be brought about using a suitable applicator device. Such a device may incorporate a hollow tube within which the sheet is accommodated when in the collapsed condition and from which it can be expelled.

The applicator device may also be used to bring about curing of the expanded sheet. The hollow tube, for example, may incorporate means for illuminating the sheet so as to transmit light energy to it.

Particularly where, as will commonly be the case, the tissue in which the puncture is formed has a substantial thickness, it may be necessary or desirable for a second sheet of material to be applied to the external surface of the tissue. Such a second sheet may have an opening by which it is mounted about the rod or stem attached to the first sheet. Again, the second sheet may be delivered using the applicator device, which is also preferably used, as for the first sheet, to initiate curing of the second sheet.

It may also be necessary or desirable for the puncture, between application of the first and second sheets, to be filled or plugged with biocompatible material, eg of collagen, fibrin or other proteinaceous material.

For some applications, in order to improve adhesion, the surfaces of the article according to the invention which, in use, are brought into contact with tissues may be coated with a layer of fluid tissue bonding material. Such a coating may take the form of a liquid or low viscosity gel, most preferably comprising the tissue-compatible bonding material in water. A certain degree of viscosity may be desirable. Viscosity-modifying components may therefore be incorporated into the composition, such as hyaluronic acid and salts thereof such as sodium hyaluronate, hydroxypropylmethylcellulose, glycerine, dextrans, honey, sodium chondroitin sulphate and mixtures thereof.

In an alternative approach intended to improve the adhesive properties of the article, the article may comprise a matrix of not only the material having tissue bonding properties but also a synthetic polymer having bioadhesive properties.

The bioadhesive polymer component of the matrix may be any polymer with suitable bioadhesive properties, ie any polymer that confers on the matrix a sufficient degree of adhesion to the tissue to which it is applied. Preferred groups of such polymers are polycarboxylic acid derivatives, a particularly preferred class of such polymers being copolymers of methyl vinyl ether and maleic anhydride, in the form of the anhydride, ester, acid or metal salt. Such polymers are supplied by International Specialty Products under the trade mark GANTREZ^{®}.

The matrix preferably further comprises a plasticiser in order to ensure that the matrix has sufficient flexibility, even after polymerisation or cross-linking. Suitable plasticisers include polyalcohols, eg glycerol, sorbitol etc.

The matrix preferably also comprises a synthetic structural polymer to confer strength and elasticity on the matrix. Suitable such polymers include water-soluble thermoplastic polymers, in particular selected from the group consisting of poly(vinyl alcohol), poly(ethylene glycol), poly(vinyl pyrrolidone), poly(acrylic acid), poly(acrylamide) and similar materials.

A relatively small proportion of surfactant, most preferably a non-ionic surfactant, will generally be incorporated into the matrix, though normally to facilitate manufacture (prevention of foaming etc) rather than to confer any beneficial property on the finished product. Suitable surfactants include block copolymers of ethylene oxide and propylene oxide, such as those sold under the trade marks Pluronic^{®} by BASF.

The matrix may be homogeneous or heterogeneous in composition, and may be of continuous or discontinuous structure. All or just some of the surface of the article may have adhesive properties.

The matrix most preferably comprises the following proportions of the individual components:
a) cross-linkable material - from about 2% to 80% by weight, more preferably 10% to 60%, and most preferably 30% to 50%;
b) structural polymer - from about 0.01 % to 20% by weight, more preferably 1% to 15%, and most preferably 2% to 10%;
c) surfactant -from about 0.001 % to 10% more preferably 0.01 % to 5%, and most preferably 0.05% to 1%;
d) plasticiser - from about 0.01% to 50%, more preferably 10% to 40%, and most preferably 20% to 40%;
e) bioadhesive polymer - from about 0.01 % to 50% by weight, more preferably 1% to 40%, and most preferably 5% to 30%.

The matrix may be manufactured by combining solutions of the different components as follows (all amounts are percentage weight of the component in the respective solution prior to combination):
a) Solution A:
   i) cross-linkable material: 5 - 60%, more preferably 10 - 40 %, and most preferably 20 to 30%.
   ii) structural polymer: 0.01 - 20%, more preferably 1 - 10% , and most preferably 2 - 8%.
   iii) surfactant : 0.001 -10%, more preferably 0.01 - 5%, and most preferably 0.1-1%.
   iv) plasticiser : 0.01 - 60%, more preferably 1 - 50%, and most preferably 10 - 40%
b) Solution B:
   i) bioadhesive polymer : 0.01 - 40%, more preferably 0.1 - 30%, and most preferably 1 - 20%.
   ii) plasticiser : 0.01 - 40%, more preferably 0.1 - 30%, and most preferably 1 - 20%

In a preferred embodiment of a sheet-like structure, where one surface only, or a selected part thereof, is bioadhesive, the matrix may be prepared by casting Solution A into a suitable non-stick mould (e.g. of PTFE), and allowing it to set through evaporation. Onto this is then cast Solution B, which is also allowed to set. During this process, the second solution penetrates into, and chemically binds to, the matrix formed by the first solution, so that the final matrix is composed of a single sheet with concentration gradients of the various components. In such a case, it will be the surface of the sheet that, in use, is brought into contact with the internal surface of the organ or vessel containing the puncture which is bioadhesive.

Alternatively, the matrix may be prepared from a single solution comprising all the components.

The casting process used to achieve the desired thickness of sheet may involve pouring, manual spreading or spraying of the component solutions.

The matrix will typically contain between 5% and 60% water by weight, and most preferably between 10% and 40%. The matrix may be partially or totally hydrated with a suitable aqueous medium at or following application (eg a body fluid or saline solution).

For some uses, it may be desirable to modify the stability of the article according to the invention - such that the half-life of the product is extended (for use in reinforcement of weakened tissue) or reduced (for drug release). This modification of stability can be effected by controlling the extent of formation of covalent bonds between molecules in the matrix (e.g. formation of disulphide bonds between protein molecules). If an increase in patch stability is desired, the matrix can be pre-treated to induce the formation of intermolecular covalent bonds.

Pre-treatment methods that can be used to modify the stability of the matrix are:
1) Heat : Temperatures from 30-70°C will promote an unravelling of the polypeptide chains, which may reduce water solubility of the protein. Exposure of the matrix to temperatures between 70°C and 120°C will promote formation of covalent bonds between albumin molecules. This will increase the stability of the article, the degree of stability achieved being dependent on the precise time, and temperature of this pre-treatment.
2) Irradiation : Electromagnetic radiation (including visible and UV light, and gamma irradiation) can promote cross-linking of albumin molecules. This is a potential method by which large articles could be pre-treated in such a way as to increase their stability.
3) Chemical : There are a large variety of chemical cross-linking reagents which could potentially be used to induce formation of covalent bonds within the matrix, including chromophore dyes such as methylene blue.

The article according to the invention or the coating (if any) of tissue bonding material applied to it may, or may not, contain a thermochromic compound (which undergoes a colour change on the application of heat) and/or a photochromic compound (which undergoes a colour change on the application of light). For example, the material may include a chromophore, such as methylene blue, which will change colour when the end point (when light activated) has been reached, as described in WO 96/22797. Such a visual colour change may provide the user with an indication that sufficient energy has been applied to ensure that curing of the tissue bonding material has occurred. In addition, when curing is complete the resultant colour change ensures that the material will absorb no further radiant energy. This provides protection against excess energy input.

If a light activated chromophore is present it provides the user, ie normally a surgeon or veterinary surgeon, with means to determine whether or not adequate energy has been provided in the desired area.

As an alternative to heat or light, curing may be brought about using a chemical activator such as a crosslinking agent, eg hexamethylenediisocyanate, which may be applied by spraying or wetting.

In some circumstances the tissue bonding material may cure spontaneously. However, it is generally preferred that curing be brought about by the application of heat or, most preferably, light.

Articles in accordance with the invention may be manufactured by various methods. A wide range of articles may be manufactured by moulding techniques, eg injection moulding using a non-cross-linked liquid, which is then cross-linked in the mould, by the application of heat or radiation. Articles in the form of solid filaments, foams and sponges may be prepared by extrusion. Such filaments may be woven or knitted into planar meshes or three-dimensional mesh shapes. Solid patches, films, foams and sponges may also be prepared by techniques such as screen printing, casting, dip-coating, injection moulding and extrusion, casting etc.

As well as methods leading to integral articles, three-dimensional articles may be fabricated from smaller components. For example, structures may be built up from sheets and/or filaments impregnated with or surrounded by liquid bonding material. Three-dimensional structures may also be built up sequentially, eg by selective curing of a bath of cross-linkable material (cf stereolithography) or by the stepwise application and curing of layers of cross-linkable material in gel form.

Articles according to the invention will generally be manufactured in the desired form and supplied as single-use, sterile devices. However, it may alternatively be possible in certain applications for the article to be constructed by the user prior to implantation. Such a case might be applicable, for instance, to scaffolds for tissue repair. In such a case, the materials supplied might include material for forming an impression of the shape to be constructed, moulding material and the material needed for formation of the final device.

The invention will now be described in greater detail, by way of illustration only, with reference to the accompanying drawings and Examples, in which
Figure 1 shows components of a first embodiment of an article according to the invention, in the form of a device for the closure of a surgical puncture;
Figure 2 shows a tip of an applicator used for applying the device of Figure 1;
Figures 3 to 5 show stages in the application of the device of Figure 1 using the applicator of Figure 2;
Figure 6 is a cut-away view of a vessel to which the device of Figure 1 has been applied;
Figure 7 is a plan view of a circular sheet of proteinaceous material forming part of a second embodiment of a device for the closure of a surgical puncture;
Figure 8 shows the device of Figure 7 in a collapsed condition; and
Figures 9 to 12 show in schematic form stages in the use of the device of Figures 7 and 8 in the closure of a surgical puncture.

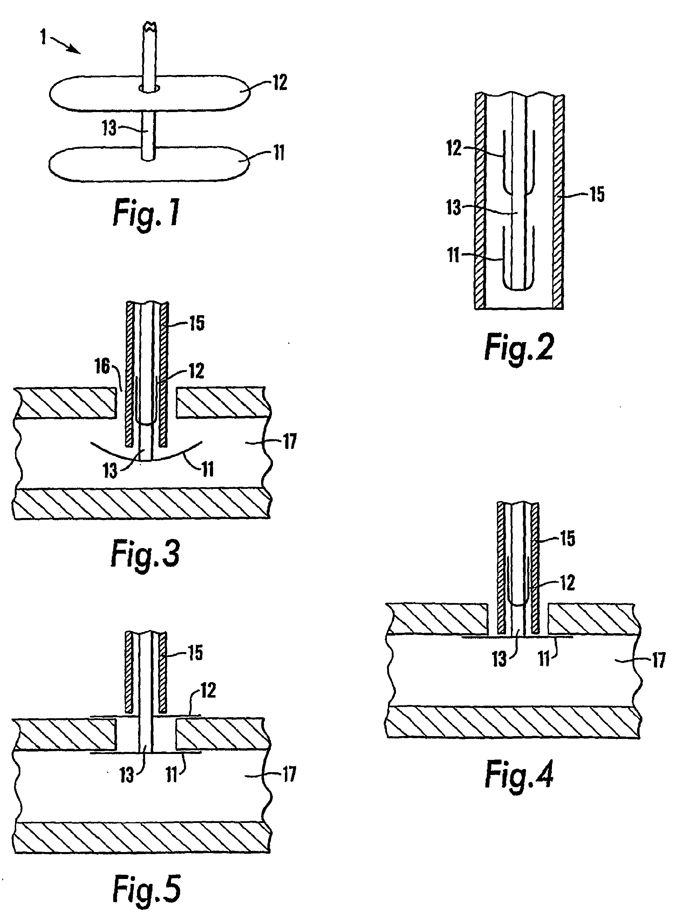

Referring first to Figure 1, a first embodiment of the invention takes the form of a device 1 for use in the closure of a surgical puncture, and comprises first and second sheets 11,12 of tissue bonding material. The first sheet 11 is fixed to one end of a solid stalk 13 of albumin-based material, the second sheet 12 having a central opening and being mounted freely about the stalk 13. The sheets 11,12 are cut from a sheet prepared by the method of one of the Examples given below.

An applicator for use in applying the device 1 of Figure 1 to a surgical puncture is illustrated schematically in Figure 2, and the manner in which the device 1 is so applied is shown schematically in Figures 3 to 5.

The applicator comprises a hollow tip 15 within which the device 1 is stored. In this condition, the sheets 11,12 are folded upwards in U-shaped configurations and spaced apart. The hollow tip 15 serves, in use, as a light guide for the application of light from a light source (not shown) to the sheets 11,12 so as to activate the sheets 11,12 and promote bonding of the sheets 11,12 to adjacent tissue, as described below.

Stages in the closure of a surgical puncture are illustrated in Figures 3 to 5, which show a puncture 16 in a vessel 17 such as an artery. First, the tip 15 is introduced through the puncture 16 and the device 1 displaced from the tip 15 sufficiently for the first sheet 11 to emerge from the end of the tip 15. Once freed from the tip 15, the first sheet 11 unfolds, as shown in Figure 3.

The tip 15 is then withdrawn through the puncture 16 sufficiently to bring the first sheet 11 into contact with the internal surface of the vessel 17 (Figure 4). Light is applied to the first sheet 11 via the tip 15 so as to activate the first sheet 11 and cause it to bond to the internal surface of the vessel 17.

Following further withdrawal of the tip 15 from the puncture 16, the second sheet 12 is released from the tip 15 and can then be pressed by the tip 15 into engagement with the external surface of the vessel 17 (Figure 5). Again, light is applied via the tip 15 to the second sheet 12 to cause it to bond to the underlying tissue.

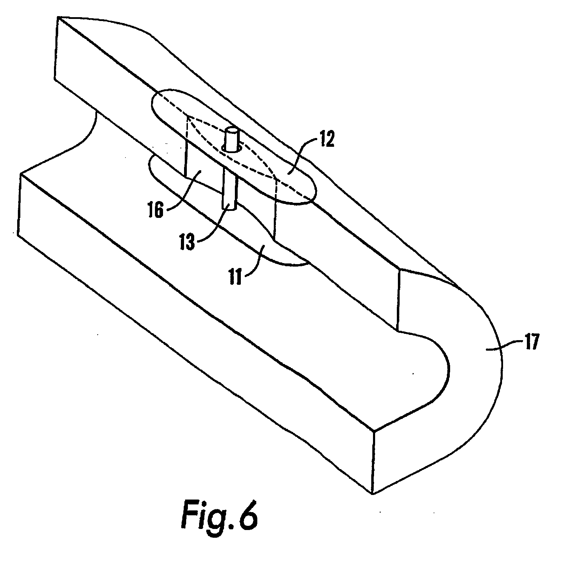

The tip 15 is retracted again and closure of the puncture 16 is completed by cutting through the stalk 13 close to the second sheet 12. The completed closure is shown in cut-away form in Figure 6.

It may be necessary or desirable for the puncture 16 to be further closed by a plug of suitable material which may be introduced after the first sheet has been bonded to the internal surface of the vessel 17. Such material may be a curable material introduced in liquid or gel form, or may be in the form of a solid or semi-solid plug which is mounted on the stalk 13, between the first and second sheets 11,12.

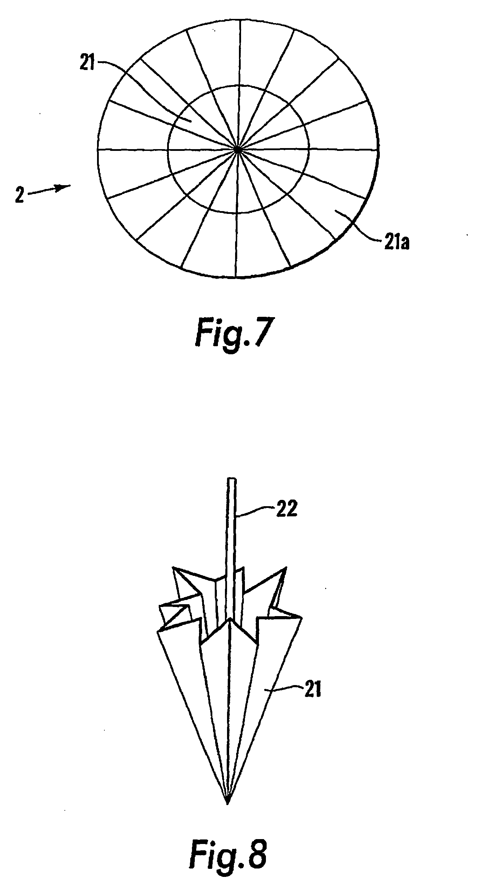

Referring now to Figures 7 to 12, a second embodiment 2 of a device according to the invention comprises a fluted circular sheet 21 of tissue bonding material to which is attached an elongate stalk 22 of solid, albumin-based material. The sheet 21 is cut from larger sheets of material prepared by the method of one of the Examples given below.

The sheet 21 is folded on the lines indicated in Figure 7 so that, after the stalk 22 has been attached to the centre of the sheet 21, it can be folded into the fluted configuration shown in Figure 8. Prior to folding in this manner, if the sheet 21 is of material that is not inherently adhesive the outer portion 21 a of the surface of the sheet 21 which, when folded into the fluted configuration, is the internal surface may be coated with a viscous albumin-containing gel having the following composition:

| | |
|---|---|
| Porcine albumin | 41% w/w |
| Methylene blue | 0.24% w/w |
| Glycerol | 2% w/w |
| Water for injection | q.s. |

The composition was made up by dissolving/dispersing the albumin, methylene blue and glycerol in the water for injection.

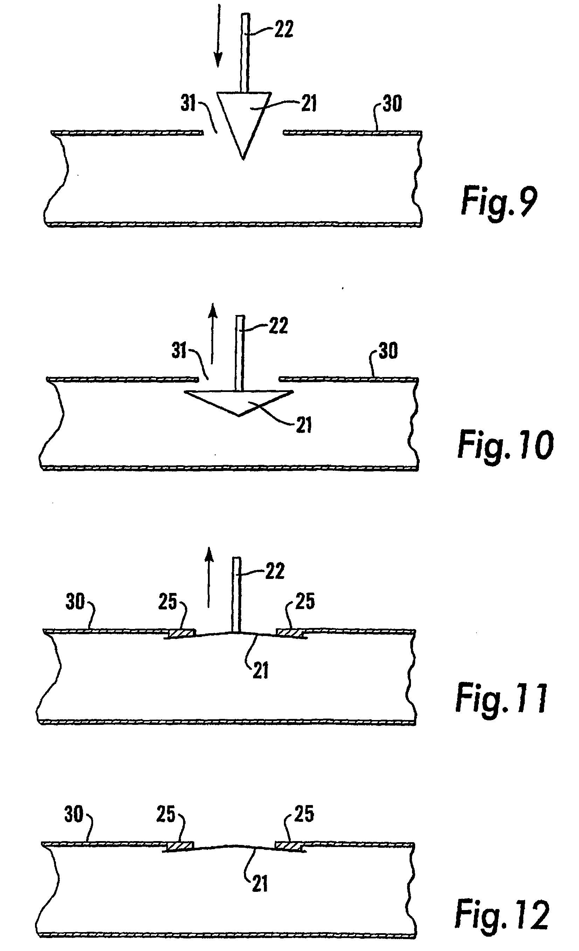

The manner in which the device 2 is used is illustrated schematically in Figures 9 to 12. Referring first to Figure 9, a vessel 30 has a puncture 31 which was formed to permit a surgical procedure and which must be closed after completion of that procedure. The vessel 30 is clamped to prevent flow of blood through the vessel 30.

The device 2 is inserted, in the collapsed condition, through the puncture 31 in the direction of the arrow in Figure 9. In the collapsed condition the overall dimensions of the fluted sheet 21 are small enough for it to pass through the puncture 31.

Once the sheet 21 is fully inserted into the vessel 30 it is drawn back by means of the stalk 22 in the direction of the arrow in Figure 10. As the device 2 is so withdrawn, the sheet 21 opens within the vessel 30 until it comes into contact with the internal surface of the vessel 30 around the periphery of the puncture 31 (see Figure 11).

Application of light of suitable intensity to the sheet 21 around the peripheral regions of the puncture 31 activates the adhesive applied to the surface of the sheet 21 and brings about the formation of bonds 25 between the sheet 21 and the tissue of the vessel 30. On completion of curing the colour changes from blue to colourless, indicating that sufficient energy has been applied.

Finally, the stalk 22 is snipped off (Figure 12) and the vessel 30 is unclamped to allow blood to flow through it once more.

Examples of the methods by which sheets of material can be prepared are as follows:

### Example 1

0.9g porcine albumin (Sigma) was dissolved in 2.5ml water for injection (Phoenix Pharmaceuticals pH 7.7) and 0.5ml of 1% w/v methylene blue for injection. To this solution, 0.585g D-sorbitol was added and dissolved. Heating of this solution in a thermostatted water bath at 59°C increases the film rehydration time from 50 seconds (if left at room temperature) to 140 seconds. This solution was left to cool for 30 minutes and then cast on a level PTFE-coated surface. The film was left to dry at room temperature for 20 hours.

### Example 2

2.84g of porcine albumin was dissolved in 9g of water for injection (Huddersfield Royal Infirmary) with 1.625g of glycerol. This solution was then used to cast sheets on a dacron (polyester) membrane. The sheet was then heated to 120°C for 10 minutes to partially crosslink the protein molecules within the sheet. This method of manufacture provided a strong sheet that was found to be insoluble in water. This method may therefore be suitable for the manufacture of sheets where long-term stability is important.

### Example 3

1.15ml of a 30% (w/w) porcine albumin solution was added to 0.2ml of glycerol and 0.125ml of polyethyleneglycol 400. This solution was used to cast sheets on a dacron (polyester) membrane. The sheets were then heated to 70°C for 30 minutes in a moist environment. Sheets prepared in this way were flexible and stretchable. These sheets may be particularly suitable for a variety of purposes.

### Example 4

1.25ml of a 30% (w/w) porcine serum albumin solution was added to 0.2ml of glycerol. This solution was used to cast sheets on a dycem non-slip membrane. The sheets were allowed to dry overnight at room temperature. The sheets were then irradiated with 3000J/cm³ ultraviolet radiation for 20 minutes. This produced a strong, stretchable sheet that was crosslinked in such a way that it would remain intact *in vivo* for an extended period of time.

### Example 5

1.51 g of porcine albumin, 0.1g of 80% hydrolysed polyvinyl alcohol, 1.42g of glycerol and 0.01 g of Pluronic 25R2 were dissolved in 2.02g of water for injection.

0.1 ml of this solution was poured onto a level PTFE surface, and spread to a thickness of approximately 50µm. The solution was heated to 120°C for 10 minutes to evaporate off water and allowed to cool.

A second solution was prepared containing 5.09g of Gantrez MS-955 and 8.5g of glycerol in 36.5g of water for injection. 0.1 ml of the second solution was similarly cast on top the cooled matrix, again to a thickness of approximately 50µm. The matrix was heated at 120°C for a further 10 minutes, and allowed to cool.

### Example 6

3.03g of porcine albumin, 0.5g of 80% hydrolysed polyvinyl alcohol, 3.00g of glycerol and 0.02g of Pluronic 25R2 were dissolved in 3.53g of water for injection. 0.1 ml of this solution was poured onto a level PTFE surface, and spread to approximately 30µm thick. The matrix was heated at 120°C for 10 minutes and allowed to cool.

0.1 ml of a second solution (from a stock comprising 5.09g of Gantrez MS-955 and 8.5g of glycerol in 36.5g of water for injection) was cast onto the cooled matrix, again to a thickness of approximately 30 µm. The matrix was heated further at 70°C for 15 minutes, and allowed to cool.

### Example 7

9.00g of porcine albumin, 1.53g of 80% hydrolysed polyvinyl alcohol, 8.98g of glycerol and 0.06g of Pluronic 25R2 were dissolved in 10.56g of water for injection. 0.3 ml of this solution was poured onto a level PTFE surface, and spread to a thickness of approximately 50µm, and left at room temperature for 1 hour.

0.3 ml of a second solution (from a stock comprising 5.09g of Gantrez MS-955 and 8.5g of glycerol in 36.5g of water for injection) was cast on top of the first matrix, again to a thickness of approximately 50µm. The matrix was left at room temperature for a further 1 hour.

### Example 8

1.51g of porcine albumin, 0.1g of 80% hydrolysed polyvinyl alcohol, 1.42g of glycerol and 0.01 g of Pluronic 25R2 were dissolved in 2.02g of water for injection. 0.1 ml of this solution was poured onto a level PTFE surface, and spread to approximately 60µm thick. The solution was heated to 120°C for 10 minutes to evaporate off water and allowed to cool.

0.1 ml of a 30% w/w Gantrez AN-119 BF (the anhydride) solution and 20% w/w glycerol, in water for injection, was similarly cast onto the existing matrix, again to a thickness of 60µm. The product was heated at 70°C for 15 minutes to evaporate water, and allowed to cool.

In each case, the sheets of material are cut to the desired shape and folded or fluted to form the non-planar structure according to the invention. The sheets prepared in accordance with Examples 1 to 4 may be coated with albumin-containing gel as described above prior to folding; the sheets prepared in accordance with Examples 5 to 8 are inherently self-adhesive.

## Claims

1. A pre-formed three-dimensional article in the form of device (1) for use in the closure of a surgical puncture, said device comprising a sheet (11,12) of material which is flexible, hydratable and capable of bonding to tissue whilst retaining its integrity, which material may be activated, leading to cross-linking of the material and the formation of chemical bonds between the material and tissue to which it is applied, and/or which material is inherently self-adhesive; said sheet being folded or collapsed to a condition such that it can be passed through the puncture into the organ or vessel in which the puncture is formed, and said sheet being adapted to expand within the organ or vessel to an operative condition in which the sheet bears against the internal surface of the organ or vessel.

2. An article as claimed in Claim 1, wherein the material has a continuous structure and performs a barrier function.

3. An article as claimed in Claim 1, wherein the material has an open structure to permit tissue ingrowth.

4. An article as claimed in any preceding claim, wherein the article is biodegradable.

5. An article as claimed in any preceding claim, which serves as a depot for the delivery of pharmacologically active compounds.

6. An article as claimed in any preceding claim, wherein the hydratable material is a crosslinkable proteinaceous or other peptide material.

7. An article as claimed in Claim 6, wherein the material comprises albumin.

8. An article as claimed in Claim 7, wherein the albumin is mammalian albumin, especially porcine albumin.

9. An article as claimed in Claim 6 or Claim 7, comprising albumin in admixture with one or more additional components.

10. An article as claimed in Claim 9, which comprises glycerol.

11. An article as claimed in Claim 1, wherein the sheet has an elongated, ovoid or rectangular shape and is folded about the lateral axis of the sheet.

12. An article as claimed in Claim 1, wherein the sheet is generally circular and is folded in a fluted configuration.

13. An article as claimed in Claim 1, wherein the sheet is attached to a stem or rod of a biocompatible material.

14. An article as claimed in Claim 13, wherein the stem or rod is of a solid proteinaceous material.

15. An article as claimed in Claim 1 in combination with an applicator device incorporating a hollow tube within which the sheet is accommodated when in the collapsed condition and from which it can be expelled.

16. An article as claimed in Claim 1, further comprising a second sheet of material applied, in use, to the external surface of the tissue.

17. An article as claimed in Claim 13 and Claim 16, wherein the second sheet of material has an opening by which it is mounted about the rod or stem attached to the first sheet.

18. An article as claimed in Claim 17, further comprising a plug of biocompatible material between the first and second sheets of material.

19. An article as claimed in Claim 1, wherein the sheet of material is 20 - 1000 µm in thickness.

20. An article as claimed in Claim 19, wherein the sheet of material is 100-500 µm in thickness.

21. An article as claimed in Claim 19, wherein the sheet comprises a single layer of material.

22. An article as claimed in Claim 19, wherein the sheet is laminated with a carrier layer of biocompatible material.

23. An article as claimed in Claim 1, wherein the surfaces of the article which, in use, are brought into contact with tissues are coated with a layer of fluid tissue bonding material.

24. An article as claimed in Claim 1, wherein the sheet comprises a matrix of not only the material having tissue bonding properties but also a synthetic polymer having bioadhesive properties.

25. An article as claimed in Claim 24, wherein the bioadhesive polymer component of the matrix is a polycarboxylic acid derivative, especially a copolymer of methyl vinyl ether and maleic anhydride, in the form of the anhydride, ester, acid or metal salt.

26. An article as claimed in Claim 24, wherein the matrix further comprises a plasticiser.

27. An article as claimed in Claim 26, wherein the plasticiser is a polyalcohol.

28. An article as claimed in Claim 24, wherein the matrix also comprises a synthetic structural polymer to confer strength and elasticity on the matrix.

29. An article as claimed in Claim 28, wherein the structural polymer is a water-soluble thermoplastic polymer, in particular selected from the group consisting of poly(vinyl alcohol), poly(ethylene glycol), poly(vinyl pyrrolidone), poly(acrylic acid) and poly(acrylamide).

30. An article as claimed in Claim 24, wherein the matrix comprises the following proportions of the individual components:
a) cross-linkable material - from about 2% to 80% by weight, more preferably 10% to 60%, and most preferably 30% to 50%;
b) structural polymer - from about 0.01 % to 20% by weight, more preferably 1% to 15%, and most preferably 2% to 10%;
c) surfactant - from about 0.001% to 10% more preferably 0.01% to 5%, and most preferably 0.05% to 1%;
d) plasticiser - from about 0.01 % to 50%, more preferably 10% to 40%, and most preferably 20% to 40%; and
e) bioadhesive polymer - from about 0.0 1 % to 50% by weight, more preferably 1% to 40%, and most preferably 5% to 30%.

31. An article as claimed in Claim 1, wherein the material contains a thermochromic compound and/or a photochromic compound.

32. An article as claimed in Claim 31, which contains a chromophore which will change colour when the material has been activated by the application of light.

33. An article as claimed in Claim 32, wherein the chromophore is methylene blue.

34. A process for the manufacture of an article as claimed in Claim 1, which process comprises forming said sheet of material by forming a film of a solution containing some or all of the components of the material, and causing or allowing the film to dry.

35. A process as claimed in Claim 34, wherein the film is formed by pouring, spreading or spraying of the solution.

36. A process for the manufacture of an article as claimed in Claim 1, which process comprises the fabrication of the article from smaller components.

37. A process as claimed in Claim 34 or 35, which process further comprises modification of the stability of the article by the application of heat, radiation or chemical agents.

## Patentansprüche

1. Vorgeformter dreidimensionaler Artikel in Form einer Vorrichtung (1) zur Verwendung beim Verschließen einer chirurgischen Punktionswunde, wobei die Vorrichtung umfasst: ein flächiges Gebilde (11, 12) aus einem Material, das flexibel, hydratisierbar und in der Lage ist, sich mit Gewebe zu verbinden, während es unversehrt erhalten bleibt, wobei das Material aktivierbar ist, was zu einer Vernetzung des Materials und der Ausbildung chemischer Bindungen zwischen dem Material und Gewebe, auf das es aufgebracht ist, führt, und/oder wobei das Material inhärent selbsthaftend ist, wobei das flächige Gebilde in einen solchen Zustand gefaltet oder zusammengelegt ist, dass es durch die Punktionswunde in das Organ oder Gefäß einführbar ist, in dem die Punktionswunde ausgebildet ist, und wobei das flächige Gebilde so angepasst ist, dass es sich in dem Organ oder Gefäß in einen operativen Zustand ausdehnt, in dem das flächige Gebilde an der Innenfläche des Organs oder Gefäßes anliegt.

2. Artikel nach Anspruch 1, wobei das Material eine kontinuierliche Struktur aufweist und eine Barrierefunktion erfüllt.

3. Artikel nach Anspruch 1, wobei das Material eine offene Struktur aufweist, um das Hineinwachsen von Gewebe zu ermöglichen.

4. Artikel nach einem vorangehenden Anspruch, wobei der Artikel biologisch abbaubar ist.

5. Artikel nach einem vorangehenden Anspruch, der als Depot für die Abgabe pharmakologisch aktiver Verbindungen dient.

6. Artikel nach einem vorangehenden Anspruch, wobei das hydratisierbare Material ein vernetzbares Eiweiß- oder anderes Peptidmaterial ist.

7. Artikel nach Anspruch 6, wobei das Material Albumin umfasst.

8. Artikel nach Anspruch 7, wobei das Albumin Säugetier-Albumin, insbesondere Albumin vom Schwein, ist.

9. Artikel nach Anspruch 6 oder Anspruch 7, der Albumin im Gemisch mit einer oder mehreren zusätzlichen Komponente(n) umfasst.

10. Artikel nach Anspruch 9, der Glycerol umfasst.

11. Artikel nach Anspruch 1, wobei das flächige Gebilde eine längliche, eiförmige oder rechteckige Form aufweist und um die Seitenachse des flächigen Gebildes gefaltet ist.

12. Artikel nach Anspruch 1, wobei das flächige Gebilde im Allgemeinen kreisförmig und in einer gekräuselten Konfiguration gefaltet ist.

13. Artikel nach Anspruch 1, wobei das flächige Gebilde an einem Schaft oder Stab aus einem biologisch abbaubaren Material befestigt ist.

14. Artikel nach Anspruch 13, wobei der Schaft oder Stab aus einem soliden Eiweißmaterial gebildet ist.

15. Artikel nach Anspruch 1 in Kombination mit einer Applikatorvorrichtung, die ein Hohlrohr einschließt, in dem das flächige Gebilde im zusammengelegten Zustand aufgenommen wird und aus dem es hinausschiebbar ist.

16. Artikel nach Anspruch 1, der ferner ein zweites flächiges Materialgebilde umfasst, das bei Verwendung auf die Außenfläche des Gewebes aufgebracht wird.

17. Artikel nach Anspruch 13 und Anspruch 16, wobei das zweite flächige Materialgebilde eine Öffnung aufweist, mit der es an dem Stab oder Schaft angebracht ist, der an dem ersten flächigen Gebilde befestigt ist.

18. Artikel nach Anspruch 17, der ferner einen Stöpsel aus biologisch abbaubarem Material zwischen dem ersten und zweiten flächigen Materialgebilde umfasst.

19. Artikel nach Anspruch 1, wobei das flächige Materialgebilde eine Dicke von 20-1000 µm aufweist.

20. Artikel nach Anspruch 19, wobei das Materialgebilde eine Dicke von 100-500 µm aufweist.

21. Artikel nach Anspruch 19, wobei das flächige Gebilde eine einzige Materialschicht aufweist.

22. Artikel nach Anspruch 19, wobei das flächige Gebilde mit einer Trägerschicht aus biologisch abbaubarem Material laminiert ist.

23. Artikel nach Anspruch 1, wobei die Oberflächen des Artikels, die bei Verwendung in Kontakt mit Geweben gebracht werden, mit einer Schicht aus flüssigem Gewebeverbindungsmaterial beschichtet sind.

24. Artikel nach Anspruch 1, wobei das flächige Gebilde eine Matrix umfasst, die nicht nur aus dem Material mit Gewebeverbindungseigenschaften, sondern auch aus einem synthetischen Polymer mit bioadhäsiven Eigenschaften gebildet ist.

25. Artikel nach Anspruch 24, wobei die bioadhäsive Polymerkomponente der Matrix ein Polycarbonsäure-Derivat, insbesondere ein Copolymer von Methylvinylether und Maleinsäueranhydrid, in Form des Anhydrids, des Esters, der Säure oder des Metallsalzes, ist.

26. Artikel nach Anspruch 24, wobei die Matrix ferner einen Weichmacher umfasst.

27. Artikel nach Anspruch 26, wobei der Weichmacher ein Polyalkohol ist.

28. Artikel nach Anspruch 24, wobei die Matrix auch ein synthetisches Strukturpolymer umfasst, um der Matrix Festigkeit und Elastizität zu verleihen.

29. Artikel nach Anspruch 28, wobei das Strukturpolymer ein wasserlösliches, thermoplastisches Polymer, insbesondere ausgewählt aus der Gruppe bestehend aus Polyvinylalkohol, Polyethylenglykol, Polyvinylpyrrolidon, Polyacrylsäure und Polyacrylamid, ist.

30. Artikel nach Anspruch 24, wobei die Matrix die folgenden Anteile der einzelnen Komponenten umfasst:
a) vernetzbares Material - ca. 2 Gew.-% bis 80 Gew.-%, bevorzugter 10 % bis 60 % und am meisten bevorzugt 30 % bis 50 %,
b) Stukturpolymer - ca. 0,01 Gew.-% bis 20 Gew.-%, bevorzugter 1 % bis 15 % und am meisten bevorzugt 2 % bis 10 %,
c) oberflächenaktives Mittel - ca. 0,001 % bis 10 %, bevorzugter 0,01 % bis 5 % und am meisten bevorzugt 0,05 % bis 1 %,
d) Weichmacher - ca. 0,01 % bis 50 %, bevorzugter 10 % bis 40 % und am meisten bevorzugt 20 % bis 40 %,
e) bioadhäsives Polymer - ca. 0,01 Gew.-% bis 50 Gew.-%, bevorzugter 1 % bis 40 % und am meisten bevorzugt 5 % bis 30 %.

31. Artikel nach Anspruch 1, wobei das Material eine thermochrome Verbindung und/oder eine photochrome Verbindung enthält.

32. Artikel nach Anspruch 31, die einen Chromophor enthält, der seine Farbe ändert, wenn das Material durch die Anwendung von Licht aktiviert wurde.

33. Artikel nach Anspruch 32, wobei der Chromophor Methylenblau ist.

34. Verfahren zur Herstellung eines Artikels nach Anspruch 1, wobei das Verfahren umfasst: Ausbilden des flächigen Materialgebildes durch Ausbilden eines Films einer Lösung, die einige oder alle der Komponenten des Materials enthält, und Trocknen oder Trocknenlassen des Films.

35. Verfahren nach Anspruch 34, wobei der Film durch Gießen, Streichen oder Sprühen der Lösung ausgebildet wird.

36. Verfahren zur Herstellung eines Artikels nach Anspruch 1, wobei das Verfahren die Herstellung des Artikels aus kleineren Komponenten umfasst.

37. Verfahren nach Anspruch 34 oder 35, wobei das Verfahren ferner die Veränderung der Stabilität des Artikels durch die Anwendung von Wärme, Strahlung oder chemischen Agenzien umfasst.

## Revendications

1. Article tridimensionnel préformé sous forme d'un dispositif (1) destiné à être utilisé dans la fermeture d'une perforation chirurgicale, ledit dispositif comprenant une feuille (11, 12) de matière qui est flexible, hydratable et capable de se lier à un tissu tout en conservant son intégrité, laquelle matière peut être activée, ce qui conduit à la réticulation de la matière et à la formation de liaisons chimiques entre la matière et le tissu auquel elle est appliquée et/ou laquelle matière est autoadhésive de manière inhérente ; ladite feuille étant repliée ou affaissée en un état tel qu'elle peut être amenée à traverser la perforation pour parvenir dans l'organe ou le vaisseau dans lequel la performation est formée, et ladite feuille étant adaptée pour s'expanser dans l'organe ou le vaisseau en un état fonctionnel dans lequel la feuille s'appuie contre la surface interne de l'organe ou du vaisseau.

2. Article selon la revendication 1 où la matière a une structure continue et remplit une fonction de barrière.

3. Article selon la revendication 1 où la matière a une structure ouverte pour permettre la croissance d'un tissu.

4. Article selon l'une quelconque des revendications précédentes où l'article est biodégradable.

5. Article selon l'une quelconque des revendications précédentes qui sert de réserve pour la délivrance de composés pharmacologiquement actifs.

6. Article selon l'une quelconque des revendications précédentes où la matière hydratable est une matière protéinique ou une autre matière peptidique réticulable.

7. Article selon la revendication 6 où la matière comprend de l'albumine.

8. Article selon la revendication 7 où l'albumine est de l'albumine de mammifère, en particulier de l'albumine porcine.

9. Article selon la revendication 6 ou la revendication 7 comprenant de l'albumine en mélange avec un ou plusieurs composants supplémentaires.

10. Article selon la revendication 9 qui comprend du glycérol.

11. Article selon la revendication 1 où la feuille a une forme ovoïde ou rectangulaire allongée, et est repliée autour de l'axe latéral de la feuille.

12. Article selon la revendication 1 où la feuille est généralement circulaire et est repliée dans une configuration striée.

13. Article selon la revendication 1 où la feuille est fixée à une tige ou barre d'une matière biocompatible.

14. Article selon la revendication 13 où la tige ou barre est en une matière protéinique solide.

15. Article selon la revendication 1 en combinaison avec un dispositif applicateur incorporant un tube creux à l'intérieur duquel la feuille est reçue quand elle est dans l'état affaissé et duquel elle peut être chassée.

16. Article selon la revendication 1 comprenant en outre une seconde feuille de matière appliquée, pendant l'utilisation, à la surface externe du tissu.

17. Article selon la revendication 13 et la revendication 16 où la seconde feuille de matière a une ouverture par laquelle elle est montée autour de la barre ou tige fixée à la première feuille.

18. Article selon la revendication 17 comprenant en outre un tampon de matière biocompatible entre les première et seconde feuilles de matière.

19. Article selon la revendication 1 où la feuille de matière a une épaisseur de 20-1 000 µm.

20. Article selon la revendication 19 où la feuille de matière a une épaisseur de 100-500 µm.

21. Article selon la revendication 19 où la feuille comprend une seule couche de matière.

22. Article selon la revendication 19 où la feuille est stratifiée avec une couche porteuse de matière biocompatible.

23. Article selon la revendication 1 où les surfaces de l'article qui, pendant l'utilisation, sont mises en contact avec des tissus sont revêtues d'une couche de matière liant les tissus fluide.

24. Article selon la revendication 1 où la feuille comprend une matrice non seulement de la matière ayant des propriétés liant les tissus mais aussi d'un polymère synthétique ayant des propriétés bioadhésives.

25. Article selon la revendication 24 où le composant polymère bioadhésif de la matrice est un dérivé de polyacide carboxylique, en particulier un copolymère de méthylvinyléther et d'anhydride maléique, sous forme de l'anhydride, ester, acide ou sel métallique.

26. Article selon la revendication 24 où la matrice comprend en outre un plastifiant.

27. Article selon la revendication 26 où le plastifiant est un polyalcool.

28. Article selon la revendication 24 où la matrice comprend aussi un polymère structural synthétique pour conférer une résistance mécanique et une élasticité à la matrice.

29. Article selon la revendication 28 où le polymère structural est un polymère thermoplastique soluble dans l'eau, choisi en particulier dans le groupe consistant en le poly(alcool vinylique), le polyéthylèneglycol, la polyvinylpyrrolidone, le poly(acide acrylique) et le polyacrylamide.

30. Article selon la revendication 24 où la matrice comprend les proportions suivantes des composants individuels :
a) matière réticulable - d'environ 2 % à 80 % en masse, de préférence encore 10 % à 60 %, et de manière particulièrement préférable 30 % à 50 % ;
b) polymère structural - d'environ 0,01 % à 20 % en masse, de préférence encore 1 % à 15 %, et de manière particulièrement préférable 2 % à 10%;
c) tensioactif - d'environ 0,001 % à 10 %, de préférence encore 0,01 % à 5 %, et de manière particulièrement préférable 0,05 % à 1 %;
d) plastifiant - d'environ 0,01 % à 50 %, de préférence encore 10 % à 40 %, et de manière particulièrement préférable 20 % à 40 % ; et
e) polymère bioadhésif - d'environ 0,01 % à 50 % en masse, de préférence encore 1 % à 40 %, et de manière particulièrement préférable 5 % à 30 %.

31. Article selon la revendication 1 où la matière contient un composé thermochromique et/ou un composé photochromique.

32. Article selon la revendication 31 qui contient un chromophore qui changera la couleur quand la matière a été activée par application de lumière.

33. Article selon la revendication 32 où le chromophore est le bleu de méthylène.

34. Procédé pour la fabrication d'un article selon la revendication 1, lequel procédé comprend la formation de ladite feuille de matière par formation d'un film d'une solution contenant certains ou tous les composants de la matière, et le séchage du film.

35. Procédé selon la revendication 34 où le film est formé par déversement, étalement ou pulvérisation de la solution.

36. Procédé pour la fabrication d'un article selon la revendication 1, lequel procédé comprend la fabrication de l'article à partir de composants plus petits.

37. Procédé selon la revendication 34 ou 35, lequel procédé comprend en outre une modification de la stabilité de l'article par application de chaleur, d'un rayonnement ou d'agents chimiques.
